# EUROPEAN PATENT SPECIFICATION

(11) **EP 0 583 024 B1**
(45) Date of publication and mention of the grant of the patent: **26.11.1997**
(21) Application number: 93202207.2
(22) Date of filing: 09.03.1989
(51) Int. Cl.: A61M 11/00, B65D 83/14, A61K 9/72, A61K 31/35, A61K 47/10

(54) **Pressurized gas dispensor for inhalationsmedicament**
Druckgasabgabevorrichtung für Inhalationsmedizin
Distributeur à gaz sous pression, pour médicament à inhaler

(30) Priority: 16.08.1988 GB 8819490
(43) Date of publication of application: 16.02.1994
(62) Divisional of application: 89302340.8
(73) Proprietor: FISONS plc, West Malling, Kent ME19 4TA (GB)
(72) Inventor: Chawla, Brindra Paul Singh, West Bridgford, Nottingham (GB)
(74) Representative: Caffin, Lee

(56) References cited:
- EP-A- 0 143 577
- EP-A- 0 260 067
- EP-A- 0 274 590
- DE-A- 3 015 813
- FR-A- 2 139 872
- FR-A- 2 339 604
- US-A- 3 024 787
- US-A- 3 131 834
- US-A- 3 985 868
- US-A- 4 696 580

## Description

This invention relates to pharmaceutical compositions, in particular compositions for administration by inhalation, packaging therefor, and the use of such compositions in the treatment of reversible obstructive airways disease.

The administration of medicaments by inhalation is well known. Medicaments for administration by the inhalation route are generally formulated either as powders, for administration by insufflation or as pressurised aerosols, or as solutions, eg aqueous solutions, for administration by nebulisation.

Solutions for inhalation may be put up either as single dose or as multi-dose formulations. Both presentations suffer from certain disadvantages. Single dose packaging is less convenient to use and more expensive and wasteful to manufacture. Such packaging frequently takes the form of glass ampoules, each containing a unit dose. Typically, the ampoule is broken open immediately prior to use and the contents transferred to a nebuliser for administration. The breaking open of the ampoule can give rise to the formation of glass sherds which are liable to be inhaled by the patient. This is clearly undesirable. Multi-dose packaging, in which the solution is packaged as a bulk from which unit doses can be dispensed immediately prior to use, is on the face of it much more attractive. However, it has hitherto been found that, in order to maintain the sterility of the solution, the formulation must contain a preservative. This is a serious disadvantage since any preservative used may *inter alia* adversely affect the stability of the solution or, more seriously, may cause unwanted side-effects. In particular, the preservative generally used, benzalkonium chloride, has been found to have unacceptable sensitising, ie allergic, effects or to have bronchoconstrictor properties when administered directly to the lung.

The present invention overcomes or substantially mitigates the above disadvantages.

According to the invention there is provided a sealed multidose dispenser provided with a one-way outlet valve and containing a pressurised gas and a sterile unpreserved aqueous solution of an inhalation medicament. The features set out in the first part of claim 1 are already apparent from EP-A-0 260 067.

The dispenser according to the invention is advantageous in that it provides a relatively inexpensive and convenient form of packaging for multi-dose pharmaceutical solutions. Furthermore, the dispenser enables the storage and multiple dosing of unpreserved solutions of medicament while maintaining adequate sterility. By "adequate sterility" we mean fewer than 50 microorganisms per gram of solution. According to one preferred aspect of the invention there is therefore provided a sealed dispenser provided with a one-way outlet valve and containing a pressurised gas and a sterile unpreserved solution of an inhalation medicament.

Aliquots of the solution contained within the dispenser may be dispensed, eg into a nebuliser for administration to a patient.

The dispenser may be manufactured from any of a number of materials. Suitable materials include, for example, glass and plastics, eg polytetrafluoroethylene. We prefer, however, that the dispenser be manufactured from metal, especially aluminium.

When the dispenser is made of metal the internal surface of the dispenser is preferably lacquered or otherwise coated to prevent or inhibit contamination of the solution with heavy metals.

The pressurised gas may be, for example, any pressurised gas suitable for use as an aerosol propellant. Examples of suitable gases are hydrocarbons, eg butanes and pentanes, nitrous oxide, carbon dioxide and dimethyl ether. We prefer, however, the pressurised gas to be nitrogen.

The dispenser may be pressurised to any pressure sufficient to bring about accurate dispensing of aliquots of solution. The pressure may, for example, be from about 30 to 100 kPa, and is typically from 50 to 80 kPa.

The one-way outlet valve is typically of conventional design and preferably includes a metering chamber. The valve is preferably crimped onto the dispenser and sealed with a gasket, eg a butyl rubber gasket. Where, as is preferred, the valve is a metering valve, the metering volume may typically be from 0.5 to 5ml. For many applications a metering volume of 2ml is appropriate.

The metering chamber of the valve preferably communicates with the interior of the dispenser via an elongate tube extending to a region near the bottom of the dispenser. This has the advantage that the dispenser may be used in the upright position. Alternatively, where no elongate tube is provided, the dispenser may be operated in the inverted position.

The valve is preferably provided with an outlet tube or spout to facilitate transfer of dispensed solution to, for example, a nebuliser.

The inhalation medicament may be of any class of drug conventionally administered by the inhalation route, eg bronchodilators, steroids and anti-allergy drugs, for example drugs which function by preventing or inhibiting the release of factors which mediate the allergic reaction. Specific drugs of the latter category which may be mentioned include those known by the generic names nedocromil sodium and sodium cromoglycate.

Generally, the solution may be prepared by methods known to be suitable for preparing solutions of the particular inhalation medicament concerned. Similarly, the dispenser may be filled by conventional methods, eg by aseptically introducing the solution into the dispenser and then pressurising under sterile conditions. Alternatively, the sterile solution may be introduced into the dispenser after pressurisation. As a further alternative, the solution and dispenser may be sterilised, eg by gamma irradiation, after filling of the solution into the dispenser and pressurisation.

The concentration of sodium cromoglycate in the solution may be in the range 0.1 to 10%, preferably from 0.5 to 5% and more preferably about 1 or 2% w/v.

The solution may also contain an effective proportion of a pharmaceutically acceptable chelating or sequestering agent such as ethylene diamine tetraacetic acid or its salts, eg its disodium salt (disodium edetate).

The concentration of the chelating or sequestering agent should be such as to ensure that no precipitate of metal salts of cromoglycic acid occurs. A suitable concentration may be from 0.01 to 1.0% w/v and preferably from 0.04 to 0.06% w/v, eg 0.05% w/v.

The formulation may also contain buffers, eg sodium dihydrogen orthophosphate (sodium acid phosphate BP), disodium hydrogen phosphate (sodium phosphate BP), sodium citrate/citric acid, and boric acid/sodium borate.

The formulation preferably has a pH in the range 3.0 to 6.0, more preferably 4.0 to 5.0.

The formulation may be made isotonic with physiological fluids by the incorporation of a suitable tonicity agent, eg sodium chloride.

An aliquot of solution dispensed from the dispenser may be administered as a nebulised cloud which may be produced by known techniques, eg using a conventional nebuliser.

The dosage to be administered will vary with the particular inhalation medicament used, and with the condition to be treated and its severity. However, in general for sodium cromoglycate a total daily dose of active ingredient in the range 15 to 30mg, eg 20mg, is satisfactory. The total daily dose may be given in 1 to 4 divided doses.

Suitable unit volumes to be administered are in the range 1 to 4ml, eg about 2ml. The unit volume is preferably administered 4 times a day or as required by the patient.

The formulations are preferably packaged in a multidose dispenser containing 10 to 300ml of solution. We prefer the formulations to be packaged in 60ml, 120ml or 240ml volumes.

The invention is illustrated, but in no way limited, by the following Examples.

### Example 1

An aerosol canister fitted with a metering valve was filled with sterile aqueous 1.0% w/v sodium cromoglycate solution and pressurised with nitrogen gas.

The aerosol canister was stored in the open laboratory for a period of 23 days. Each day a 1g sample of the solution from the canister was weighed into a 10ml aliquot of sterile peptone water, filtered through a sterile membrane filter, washed with 100ml USP phosphate buffer pH 7.2 and transferred onto either tryptone soya agar (for bacteria) or malt extract agar (for yeasts and moulds).

Bacterial plates were incubated at 30°C for 3 days and the fungal plates were incubated at 25°C for 7 days. Following incubation any microorganisms growing on the filters were counted.

The results were as follows:

### a) Bacterial contamination

2 bacteria were counted in the sample taken on day 21 and 1 in the sample from day 9. Otherwise no microorganisms were observed.

### b) Yeast and Mould contamination

1 microorganism was counted in the samples from day 8, day 21 and day 22. Otherwise no microorganisms were observed.

These results indicate that over the three weeks of testing there was no proliferation of any microorganisms which may have contaminated the sodium cromoglycate solution.

## Claims

1. A sealed multidose dispenser provided with a one-way outlet valve and containing a pressurised gas and an inhalation medicament, characterised in that the inhalation medicament is in the form of a sterile unpreserved aqueous solution.

2. A dispenser according to Claim 1, which is manufactured from aluminium lacquered or otherwise coated to prevent or inhibit contamination of the solution.

3. A dispenser according to any one of the preceding claims, wherein the pressurised gas is nitrogen.

4. A dispenser according to any one of the preceding claims, pressurised to a pressure of from 30 to 100 kPa.

5. A dispenser according to any one of the preceding claims, wherein the one-way outlet valve is a metering valve with a metering volume of from 0.5 to 5ml.

6. A dispenser according to any one of the preceding claims, wherein the inhalation medicament is an anti-allergy drug which functions by preventing or inhibiting the release of factors which mediate the allergic reaction.

7. A dispenser according to claim 6, wherein the anti-allergy drug is nedocromil sodium or sodium cromoglycate.

## Patentansprüche

1. Versiegelter Mehrfachdosis-Spender, welcher mit einem Einweg-Auslaßventil versehen ist sowie ein unter Druck stehendes Gas und ein Inhalationsmedikament enthält, dadurch gekennzeichnet, daß das Inhalationsmedikament in Form einer sterilen nichtkonservierten wässerigen Lösung vorliegt.

2. Spender nach Anspruch 1, welcher aus Aluminium hergestellt ist, das lackiert oder auf andere Weise beschichtet ist, um eine Verunreinigung der Lösung zu verhindern oder zu inhibieren.

3. Spender nach einem der vorhergehenden Ansprüche, wobei das unter Druck stehende Gas Stickstoff ist.

4. Spender nach einem der vorhergehenden Ansprüche, welcher unter einen Druck von 30 bis 100 kPa gesetzt ist.

5. Spender nach einem der vorhergehenden Ansprüche, wobei das Einweg-Auslaßventil ein Dosierventil mit einem Dosiervolumen von 0,5 bis 5 ml ist.

6. Spender nach einem der vorhergehenden Ansprüche, wobei das Inhalationsmedikament ein antiallergisches Arzneimittel ist, das durch die Prävention oder Inhibition der Freisetzung von die allergische Reaktion vermittelnden Faktoren wirkt.

7. Spender nach Anspruch 6, wobei das antiallergische Arzneimittel Nedocromil-Natrium oder Natrium-Cromoglykat ist.

## Revendications

1. Distributeur multidose étanche comportant une valve de décharge antiretour et contenant un gaz sous pression et un médicament à inhaler, caractérisé en ce que le médicament à inhaler se présente sous la forme d'une solution aqueuse stérile sans agents de conservation.

2. Distributeur suivant la revendication 1, fabriqué en aluminium, verni ou recouvert d'une autre manière pour éviter ou empêcher la contamination de la solution.

3. Distributeur suivant l'une quelconque des revendications précédentes, dans lequel le gaz sous pression est de l'azote.

4. Distributeur suivant l'une quelconque des revendications précédentes, maintenu à une pression de 30 à 100 kPa.

5. Distributeur suivant l'une quelconque des revendications précédentes, dans lequel la valve de décharge antiretour est une valve de dosage ayant un volume de dosage compris entre 0,5 et 5 ml.

6. Distributeur suivant l'une quelconque des revendications précédentes, dans lequel le médicament à inhaler est un antiallergique dont le rôle est d'éviter ou d'empêcher le déclenchement de facteurs qui favorisent la réaction allergique.

7. Distributeur suivant la revendication 6, dans lequel l'antiallergique est du nédocromil sodique ou du cromoglycate sodique.
